## Europäisches Patentamt

## European Patent Office

## Office européen des brevets

(11) Publication number: **0 010 988**
**B1**

(12) **EUROPEAN PATENT SPECIFICATION**

(45) Date of publication of patent specification: 30.05.84

(21) Application number: **79302446.4**

(22) Date of filing: **05.11.79**

(51) Int. Cl.³: **G 01 N 33/24,** G 01 N 3/00,
G 01 N 3/40, G 01 N 3/42,
G 01 N 19/00, G 01 N 19/02,
E 21 C 39/00, E 02 D 1/00

(54) Electrical friction sleeve cone penetrometer.

(30) Priority: **06.11.78 AU 6671/78**

(43) Date of publication of application:
**14.05.80 Bulletin 80/10**

(45) Publication of the grant of the patent:
**30.05.84 Bulletin 84/22**

(84) Designated Contracting States:
**AT BE CH DE FR GB IT LU NL SE**

(56) References cited:
FR-A- 983 514
GB-A-1 102 731
GB-A-1 215 417
GB-A-1 453 273
US-A-3 956 924
US-A-3 988 923

J. REIMPELL, W. BACHMANN "Handbuch des
Waagenbaues, Band 3, Elektromechanische
Waagen", 1966, B.F. Voigt Verlag Handwerk
und Technik, Berlin-Hamburg, pages 65, 66, 67

(73) Proprietor: MacGregor, John Stuart
Town House 5 76 Molesworth Street
Kew Victoria 3101 (AU)

(72) Inventor: MacGregor, John Stuart
Town House 5 76 Molesworth Street
Kew Victoria 3101 (AU)

(74) Representative: Eyles, Christopher Thomas et al
BATCHELLOR, KIRK & EYLES 2 Pear Tree Court
Farringdon Road
London, EC1R 0DS (GB)

(56) References cited:
K. HOFFMANN "Grundlagen der
Dehnungsmessstreifen - Technik,
Massnahmen zur Verweidung bzw.
Verminderung von Messfehlern", 1978,
HOTTINGER BALDWIN MESSTECHNIK,
DARMSTADT, pages 3 to 7

K. HOFFMANN "Grundlagen der
Dehnungsmessstreifen - Technik: Eine
Einführung in die Dehnungsmessstreifen -
Technik anhand von
Versuchsbeschreibungen", 1976, HOTTINGER
BALDWIN MESSTECHNIK, DARMSTADT,
pages 23-25, 37-39

Courier Press, Leamington Spa, England.

## Description

This invention relates to an improved quasi-static electrical friction sleeve cone penetrometer.

A penetrometer is a device for measuring the penetrability of semisolids in general, and soil in particular. The first penetrometer was produced by Collin in France in 1846, and for more than 100 years penetrometers were mechanical devices of increasingly sophistication which included a probe portion which was pushed or driven into the ground, which produced by mechanical means an indication of the resistance of the soil to the probe.

There have developed two basic types of penetrometers, dynamic and static (actually quasi-static). This invention is concerned with quasi-static penetrometers, which are advanced into the ground at a substantially constant velocity, and produce cone resistance readings at various depths, and in particular with electric quasi-static friction sleeve cone penetrometers which have a cone for measurement of cone resistance, and a friction sleeve for measurement of side friction, and in which the measured quantities are transmitted electrically from those elements and displayed on electrically operated equipment.

Present penetrometers are most commonly used to define the natural soil profile or stratigraphy at a site. A knowledge of the geology of an area, combined with penetrometer results, enables the soil profile across the site to be determined. The description of each layer is obtained from characteristic patterns of the cone resistance (R), side friction (F), and friction ratio (F/R).

This information is an essential prerequisite to any major building construction or civil engineering project at a particular site, the only alternatives, in situ load tests on full scale foundations, laboratory test on indisturbed soil samples and in situ testing of soils, being slow and expensive.

One known type of prior quasi-static friction sleeve cone penetrometer is the Fugro, which dates from 1970, and is described in The Penetrometer and Soil Exploration by G. Sanglerat, Elsevier, 1972.

The Fugro consists of a probe which is connectible to pushing rods which are used to advance the probe into the ground from a drilling rig, usually mounted with the ancillary equipment for recording and displaying information from the probe, in a heavy motorized vehicle.

The probe consists of an adaptor for connection to the pushing rods, and a body which contains two load cell portions, one of which is connected to the cone, and the other of which is connected to the friction sleeve. Strain gauges are provided on each load cell, and wiring connected to the strain gauges is run through a cable to the recording and displaying equipment.

It should be appreciated that F is much smaller than R, in the vicinity of $10^{-3}R$, and accordingly it is essential that the smaller quantity be accurately measured. In the Fugro, F is determined by measuring F + R, and then by measuring R, and subtracting R from F + R to find F. As R and F + R are relatively large quantities, the accuracy of the value of F produced by subtraction of one of those quantities from the other is not great.

In addition the Fugro suffers from structural weaknesses in the body and the friction sleeve, the latter being connected to the body at only one point, and being subjected to lateral forces from the soil and rocks and the like encountered during advancement of the probe. The Fugro also uses mains power operated equipment in the large vehicle, which makes the setting up of a Fugro at a site a complex and costly exercise.

Another known form of penetrometer is that shown in patent GB—A—1,102,731. This comprises a main tube with a central bore, through which bore the electrical connections to the strain gauges may be led. At the lower end a screw thread is provided by which an extension tube is screwed to the main tube, the extension tube being connected by means of a force measuring element to a conical tip.

At a short distance above the screw connection the tube is provided with a slot through which a bridge piece extends, the extremities of the bridge piece abutting on a shoulder of a jacket or sleeve. This jacket has the same outer diameter as the extension tube and fits with a relatively large clearance around the lower end of the tube. The bridge piece is in its central part provided with a cylindrical or spherical seat, in which seat a complementary hemispherical surface of a measuring rod is received, the measuring rod being provided with strain gauges in order to measure its deformation. At the other extremity the measuring rod has a hemispherical surface which fits in a complementary seat in a bridge fixed in the bore of the main tube.

The adhesion forces being exerted by the surrounding soil on the jacket are transmitted by the shoulder to the bridge piece and further to the measuring rod, the latter being supported by the bridge so that it will be compressed.

This type of construction also suffers from a number of disadvantages. The axial mounting of the measuring rod which comprises the friction load cell on a separate bridge piece at its lower end, and the mounting of this bridge piece on shoulders on the inside surface of the outer jacket or sleeve, increases the possibility that the device will bind up or jam under the influence of high lateral forces. Since the load cells are not integral with, or positively connected to the main body and the document does not show how the various parts can be made to positively cooperate with one another, it would also be difficult to construct a device of this type in such a way as to make it sufficiently rigid, and therefore reliable, for the usually demanding conditions of use.

Accordingly, the present invention provides a quasi-static cone penetrometer including a generally cylindrical probe having a tubular main body, a cone for measuring the resistance of a medium into which the probe is advanced, and a friction sleeve for measuring the side friction of said medium, a cone load cell connected to said cone, and a friction load cell connected to said friction sleeve and to the main body, the cone load cell and the friction load cell each having transducers attached thereto to respectively convert actual cone resistance and side friction into electrical signals to provide a separate measurement of each quantity, characterised in that the cone load cell is integral with the tubular main body and the friction load cell comprises a tubular member which is coaxially positioned between the friction sleeve and the tubular main body and which has one end portion connected to the said friction sleeve whilst the other end portion is connected to the tubular main body.

The elements of the penetrometer probe may be fabricated from steel using conventional metalworking techniques.

An embodiment of the invention will be described in detail hereinafter, with reference to the accompanying drawings, in which:

Figure 1 is a sectioned elevation of an assembled penetrometer probe, with the strain gauge and wiring details omitted;

Figure 2 is a partly-sectioned perspective view of the friction sleeve of the probe;

Figure 3 is a partly-sectioned perspective view of the main body and cone load cell of the probe;

Figure 4 is a partly-sectioned perspective view of the friction sleeve load cell of the probe; and

Figure 5 is a block diagram of circuitry of the penetrometer, located within the probe and externally of the probe.

This embodiment of the invention is described in relation to the primary application of penetrometers, soil analysis and exploration. However, it must be stresssed that the penetrometer of this embodiment, as a result of the accuracy of its results, may be used in the analysis of any resistive medium, such as cheese, soil conservation, detection of minerals in beach sands, forestry, ship design, archaeology, viscous fluid studies, aeronautical studies and agriculture generally.

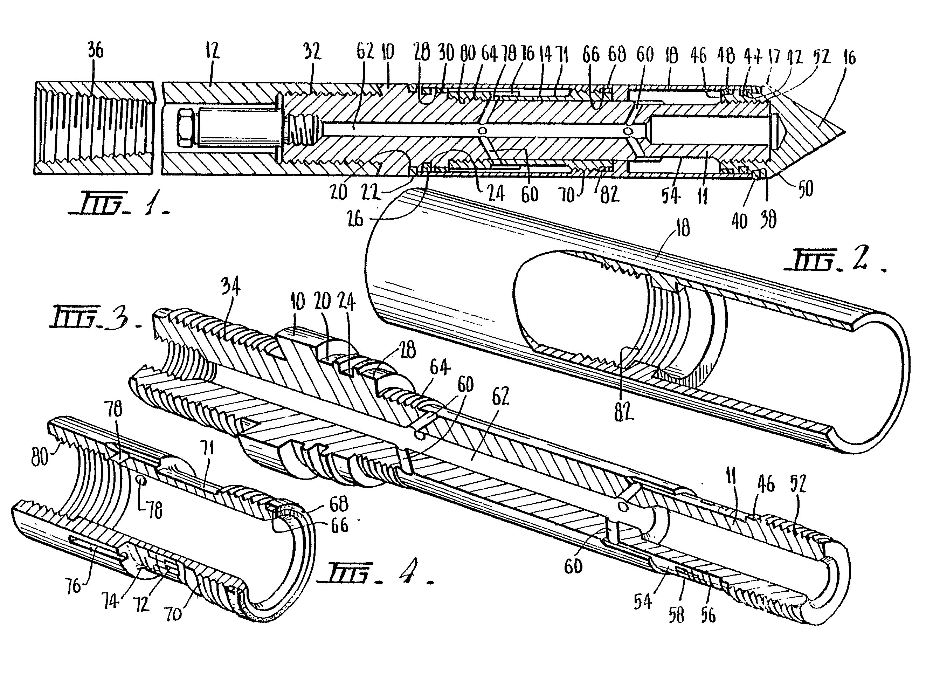

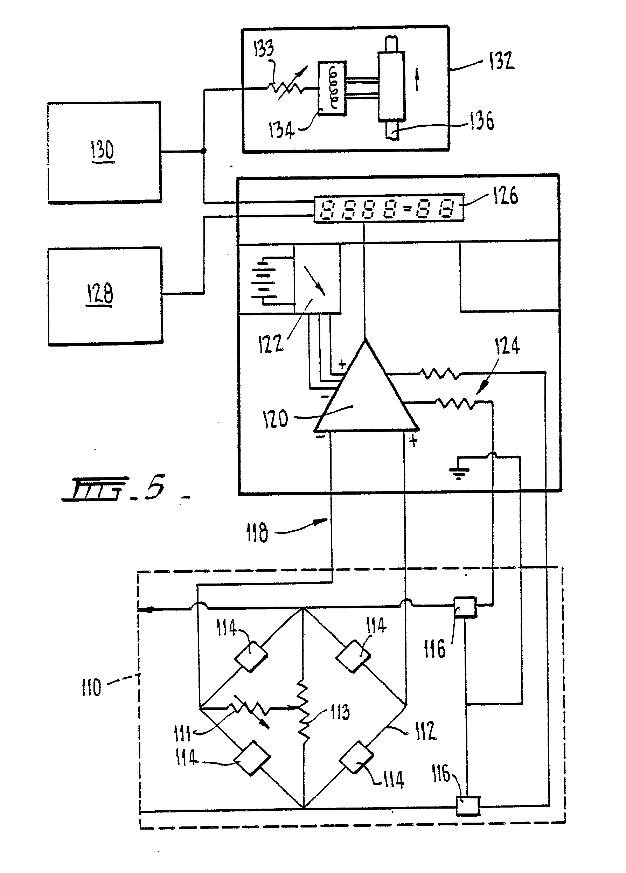

Referring firstly to Figure 1, there is shown a sectioned assembled penetrometer probe according to this invention. The probe consists of a main body and cone load cell 10 (shown in more detail in Figure 3), to which is secured an adaptor 12, the elements 10 and 12 being screwed together by means of co-operating screwthreads 32. The adaptor 12 is provided with a tapering internally screwthreaded portion 36 into which the lowermost rod of a series of pushing rods (not shown, but referred to later) is screwed.

Towards the 'adaptor' end of body 10, there are provided annular grooves 20, 24, and a recessed annular shoulder 28. In these grooves there are seated respectively, O-rings 22 and 26, and an annular sleeve 30. Located next to the shoulder 28, in an externally screwthreaded portion 64 (Figure 3) of a diameter less than that of the shoulder. The remote end of the body is a cone load cell 11, and consist of a wide annular recess 54, and an externally threaded portion 52, to which the cone 16 may be screw-fitted.

A friction sleeve load cell 14 (Figure 4) is screwed to body 10, by the cooperation of load cell internal screwthread 80, and external body screwthread 64. The cell 14 is securely attached to the body 10 by a grub screw located in an internally screwthreaded aperture 78. Load cell 14 has a wide annular recess 71, and an externally screwthreaded portion 70 at the end opposite to that which is screw-fitted to body 10. An annular recessed shoulder 66 is provided at this end of the cell 14, and a sleeve 68 is sealed in the shoulder.

A friction sleeve 18 is secured to load cell 14 by the cooperation of the sleeve internally threaded portion 82 and load cell externally threaded portion 70. Finally, to complete the assembly of elements in the penetrometer probe, the cone 16 is screwed onto externally threaded portion 52 of body 10.

Cone 16 has annular grooves 38, 42, and a recessed annular shoulder 46, the reverse of grooves and shoulder 20, 24 and 28. Seated in the grooves, respectively, are O-rings 40 and 44, and seated on the shoulder 46 is an annular sleeve 48.

It can be seen that when friction sleeve 18 is screwed onto load cell 14, the inner end rides over sleeve 30 and O-ring 26, and abuts O-ring 22. Similarly, when cone 16 is screwed onto body 10, sleeve 48 and O-ring 44 ride under friction sleeve 18, and O-ring 40 abuts the outer end of sleeve 18.

Preferably, the O-rings are formed from a tough elastomeric material, and the sleeves 48, 68 and 30 are all formed from polytetrafluoroethylene. The sleeves may be glued into place with an epoxy resin or glue. The first material provides for the absorption of shocks and limited movement, and the second material, as used in a relatively thin annular sleeve, is extremely strong, and the three sleeves thus resist lateral forces on the friction sleeve 18, which can distort sleeve friction readings. The use of thin sleeves also eliminates the need for deep grooves to seat thicker sleeves, and thus the elements of the probe are strengthened.

Apart from the O-rings and sleeves, and the strain gauges, printed circuits, balancing bridge, and cabling (to be described hereinafter) the penetrometer probe elements are preferably made from a strong, hard steel. The friction sleeve 18 is preferably of tempered steel having a hardness of between 45 and 48 Rockwell C. The cone 16 is preferably manganese tool steel, oil quenched and tempered. The strength of the cone and sleeve is an important factor, as the cone and sleeve contain the material-contacting surfaces, and weakness therein can lead to faulty results.

The cone 16 has a standard cone angle of 60°, and a conical surface area of 10 square centimetres. The cone also has a shoulder portion 17, which allows some wear of the cone surface, without allowing distortion of the friction sleeve. The friction sleeve has a cylindrical surface of 150 square centimetres. The penetrometer probe construction of Figures 1 to 4 enables the probe to be resistant to lateral forces on the sleeve, impacts on the cone, and bending of the main body of the probe.

The load cells 11 and 14, being connected to cone 16 and friction sleeve 18 respectively, enable simultaneous and distinct measurement of cone resistance and side friction to take place. In order to quantify these measurements, transducers in the form of strain gauges are affixed to each load cell.

On load cell 11, six double strain gauges 58 are secured to surface 54 by an epoxy glue or the like. The strain gauges, each of which has portions to measure axial and tangential stresses, are arranged symmetrically around the surface. A flexible printed circuit strip 56 is also secured to and around surface 54. The strip has staggered conductors in two rows parallel to the longitudinal axis of the strip each row being interrupted, the interrupted portions of each row being staggered. The gauges are electrically connected to the printed circuit, and from there the wiring is taken through an aperture 60 to the hollow central section 62 of body 10 and cell 11. In this hollow section, balancing bridge and associated circuitry (not shown in Figure 3, but to be described hereinafter) is located, so that the temperature of the cell affects the strain gauges and the balancing circuitry. From the circuitry, the wiring extends through the hollow centre 62 into a cable (not shown) which extends through adaptor 12 and the pushing rods to the display and ancillary equipment, to be described hereinafter.

Load cell 14 has strain gauges 72 and printed circuit 74 attached to the surface 71 in a similar manner; and the circuitry and wiring associated with the gauges is parallel to that of the cone load cell, the wiring from the printed circuit extending through aperture 76 to the hollow interior of the load cell.

In operation, the penetrometer probe of Figures 1 to 4 is pushed into the ground by a drilling rig or similar apparatus, usually hydraulically operated. The rig operates to push the pushing rods, the lowermost of which is screwed to the adaptor 12, at a carefully controlled constant rate of, preferably, 2 cm/sec. The rods are screwthreaded, internally at one end, and externally at the other, so that they may be screwed together to form a continuous rod structure which can extend to a distance of 45 m. It is desirable to use a rig which has a stable platform from which to operate. Stability and careful control of probe velocity significantly increase the accuracy of results obtained in any single test.

Figure 5 is a block diagram of the probe strain gauge and associated circuitry and electrical equipment, which is advantageously all battery operated with rechargeable batteries. The rectangle 110 depicted in broken lines contains the electrical equipment included within one load cell. The other load cell equipment is identical, and accordingly need not be shown.

Equipment 110 includes a balancing bridge 112 which incorporates the strain gauges 114 of a load cell, a balancing potentiometer 113, and an additional potentiometer 111, which acts as a vernier adjustment to the potentiometer 113. This enables accurate balance to be undertaken within the probe, such that minimum balancing needs to be carried out at the display equipment end. Voltage regulators 116 are provided in the circuit.

The wiring from equipment 110 travels through cable 118, to amplifier 120, which is associated with a balancing net 124 and a high quality voltage regulator 122. The signal from equipment 110 is fed to a digital display 126, preferably a liquid crystal display, which provides a continuous calibrated quantitative visual readout of the cone resistance or the side friction. In practice, due to the difference in order of the quantities involved, cone resistance R is displayed in MPa, and side friction R in KPa. The signal to the display can also be fed to a microprocessor 128, for further immediate soil analysis. The sinal can also be fed to a chart recorder 130, for a continuous 'hard copy' record of the measured quantities. In practice, graphs are produced of cone resistance (R), side friction (F), and friction ratio (F/R), each as a function of probe depth.

Block 132 contains circuitry which also receives the signal from the particular load cell; the circuitry operates to stop the rod pushing operation once either of the measured quantities exceeds a predetermined value, thus preventing damage to the probe.

An example is shown within block 132, where a potentiometer 133 is used to set a predetermined maximum signal level; a relay 134 is actuated when that level is reached. The relay, when actuated, acts to switch off a pushing machine, or as shown, closes a valve in the hydraulic system, part of which is designated by reference numeral 136.

## Claims

1. A quasi-static cone penetrometer including a generally cylindrical probe having a tubular main body (10), a cone (16) for measuring the resistance of a medium into which the probe is advanced, and a friction sleeve (18) for measuring the side friction of said medium, a cone load cell (11) connected to said cone, and a friction load cell (14) connected to said friction sleeve (18) and to the main body, the cone load cell (11) and the friction load cell (14) each having transducers attached thereto to respectively convert actual cone resistance and side friction into electrical signals to provide a separate measurement of each quantity, characterised in that the cone load cell (11) is integral with the tubular main body (10) and the friction load cell (14) comprises a tubular member which is coaxially positioned between the friction sleeve (18) and the tubular main body (10) and which has one end portion (70) connected to the said friction sleeve (18) whilst the other end portion (80) is connected to the tubular main body (10).

2. A penetrometer according to claim 1 further characterised in that each of said transducers includes a series of strain gauges (114) bonded to the external surface of the respective load cell (11, 14), said strain gauges being electrically connected to a flexible printed circuit bonded to said external surface, said printed circuit having two parallel rows of conductors, each row being interrupted, the interrupted portions of each row being staggered.

3. A penetrometer according to claim 2 further characterised in that each of said transducers further includes a bridge network, incorporating said strain guages and said printed circuit, the network including a balancing potentiometer (113) and a vernier potentiometer (111) on the movable arm of the balancing potentiometer.

4. A penetrometer according to claim 2 or claim 3 further characterised in that there is provided an amplifier (120) for amplifying the signals from the strain gauges, a digital display (126) for quantitatively displaying the quantity measured by said strain gauges, and a chart recorder (130) for recording cone resistance R, side friction F and friction ratio F/R.

5. A penetrometer according to claim 4, further characterised in that cut-off means (132) are provided, operative when one of said quantities reaches a predetermined value, to prevent further advancement of the penetrometer probe.

## Patentansprüche

1. Quasistatisches Kegel-Penetrometer, mit einer im allgemeinen zylindrischen Sonde, die einen rohrförmigen Hauptkörper (10) hat, einem Kegel (16) zur Messung des Widerstands eines Mediums, in welches die Sonde bewegt wird, und einer Reibungshülse (18) zur Messung der Seitenreibung des genannten Mediums, einer Kegellastzelle (11), die mit dem Kegel verbunden ist, und einer Reibungslastzelle (14), die mit der genannten Reibungshülse (18) und mit dem Hauptkörper verbunden ist, wobei an der Kegellastzelle (11) und der Reibungslastzelle (14) jeweils Wandler befestigt sind, um den tatsächlichen Kegelwiderstand und die Seitenreibung in elektrische Signale zu wandeln, um eine separate Messung für jede Größe zu liefern, dadurch gekennzeichnet, daß die Kegellastzelle (11) einstückig mit dem rohrförmigen Hauptkörper (10) ausgebildet ist und die Reibungslastzelle (14) ein rohrförmiges Teil umfaßt, welches koaxial zwischen der Reibungshülse (18) und dem rohrförmigen Hauptkörper (10) angeordnet ist und dessen einer Endabschnitt (70) mit der genannten Reibungshülse (18) verbunden ist, während dessen anderer Endabschnitt (80) mit dem rohrförmigen Hauptkörper (10) verbunden ist.

2. Penetrometer nach Anspruch 1, ferner dadurch gekennzeichnet, daß jeder der genannten Wandler eine Reihe von Dehnungsmessern (114) umfaßt, die mit der äußeren Oberfläche der jeweiligen Lastzelle (11, 14) verbunden sind, und die elektrisch mit einer flexiblen gedruckten Schaltung verbunden sind, die an der genannten äußeren Oberfläche befestigt ist und zwei parallele Reihen von Leitern umfaßt, von denen jede Reihe unterbrochen ist, wobei die unterbrochenen Abschnitte jeder Reihe gestaffelt sind.

3. Penetrometer nach Anspruch 2, ferner dadurch gekennzeichnet, daß jeder der genannten Wandler ferner ein Brückennetz umfaßt, welches die genannten Dehnungsmesser und die genannte gedruckte Schaltung enthält, und das Netz ein Abgleichpotentiometer (113) und ein Feineinstellungspotentiometer (111) auf dem beweglichen Arm des Abgleichpotentiometers umfaßt.

4. Penetrometer nach Anspruch 2 oder 3, ferner dadurch gekennzeichnet, daß ein Verstärker (120) zur Verstärkung der Signale von den Dehnungsmessern, eine digitale Anzeige (126) zur quantitativen Anzeige der von den genannten Dehnungsmessern gemessenen Größe und ein Blattschreiber (130) zur Aufzeichnung des Kegelwiderstands R, der Seitenreibung F und des Reibungsverhältnisses F/R vorgesehen sind.

5. Penetrometer nach Anspruch 4, ferner dadurch gekennzeichnet, daß Abschalteinrichtungen (132) vorgesehen sind, die tätig werden, wenn eine der genannten Größen einen vorbestimmten Wert erreicht, um einen weiteren Vorschub der Penetrometersonde zu verhindern.

## Revendications

1. Pénétromètre quasi-statique à cône incluant une sonde pratiquement cylindrique ayant un corps principal tubulaire (10); un cône (16) pour mesurer la résistance d'un milieu dans lequel on fait avancer la sonde, et un manchon de friction (18) pour mesurer la friction latérale dudit milieu, une cellule dynamométrique (11) reliée audit cône, et une cellule dynamométrique de friction (14) reliée audit manchon de friction (18) et au corps principal, la cellule dynamométrique (11) du cône et la cellule dynamométrique de friction (14) comportant chacune des transducteurs pour convertir respectivement la résistance réelle rencontrée par le cône et la friction latérale en signaux électriques afin de fournir une mesure séparée de chaque quantité, caractérisé en ce que la cellule dynamométrique (11) du cône fait partie intégrante du corps principal tubulaire (10) et en ce que la cellule dynamométrique de friction (14) comprend un organe tubulaire qui est disposé coaxialement entre le manchon de friction (18) et le corps principal tubulaire (10) et qui a une extrémité (70) reliée audit manchon de friction (18), tandis que son autre extrémité (80) est reliée au corps principal tubulaire (10).

2. Pénétromètre selon la revendication 1, caractérisé, en outre, en ce que chacun desdits transducteurs comprend une série de jauges de contrainte (114) collées à la surface extérieure de la cellule dynamométrique correspondante (11, 14), lesdites jauges de contrainte étant reliées électriquement à un circuit imprimé flexible collé à ladite surface extérieure, ledit circuit imprimé comportant deux rangées parallèles de conducteurs, chaque rangée étant interrompue, les parties interrompues de chaque rangée étant décalées ou échelonnées.

3. Pénétromètre selon la revendication 2, caractérisé en ce que chacun desdits transducteurs comporte également un circuit en pont, dans lequel sont incorporées lesdites jauges de contrainte et ledit circuit imprimé, ce circuit incluant un potentiomètre d'équilibrage (113) et un potentiomètre de réglage fin (111) sur le curseur du potentiomètre d'équilibrage.

4. Pénétromètre selon la revendication 2 ou 3, caractérisé en ce qu'il comporte un amplificateur (120) pour amplifier les signaux des jauges de contrainte, un dispositif d'affichage (126) pour afficher la valeur numérique de la quantité mesurée par lesdites jauges de contrainte, et un enregistreur à bande (130) pour enregistrer la résistance R rencontrée par le cône, la friction latérale F et le rapport des frictions F/R.

5. Pénétromètre selon la revendication 4, caractérisé en ce qu'il comporte des moyens de coupure (132) qui interviennent quand l'une desdites quantités atteint une valeur prédéterminée, pour empêcher la sonde du pénétromètre de continuer à avancer.

FIG. 1.

FIG. 2.

FIG. 3.

FIG. 4.

0010988

FIG.5